# EUROPEAN PATENT SPECIFICATION

(11) **EP 0 393 850 B1**
(45) Date of publication and mention of the grant of the patent: **07.01.1993**
(21) Application number: 90303044.3
(22) Date of filing: 21.03.1990
(51) Int. Cl.: A23J 1/20, C07K 3/26

(54) **Process for producing kappa-casein glycomacropeptides**
Verfahren zur Herstellung von kappa-Casein-Glykomakropeptiden
Procédé de préparation de glycomacropeptides de kappa-caséine

(30) Priority: 19.04.1989 JP 97583/89
(43) Date of publication of application: 24.10.1990
(73) Proprietor: SNOW BRAND MILK PRODUCTS CO., LTD., Sapporo-shi, Hokkaido 065 (JP)
(72) Inventor: Tanimoto, Morimasa, Sayama-shi, Saitama-ken (JP); Kawasaki, Yoshiro, Yukijirushi Nyugyo K.K., Kawagoe-shi, Saitama-ken (JP); Shinmoto, Hiroshi, Kawagoe-shi, Saitama-ken (JP); Dosako, Shunichi, Urawa-shi, Saitama-ken (JP); Tomizawa, Akira, Sayama-shi, Saitama-ken (JP)
(74) Representative: Ackroyd, Robert

(56) References cited:
- EP-A- 0 291 264

## Description

The present invention relates to processes for simply producing kappa-casein glycomacropeptides which have several kinds of physiological activities.

It has been known that kappa-casein glycomacropeptides are peptide-bonded sialic acids which are produced by reacting milk kappa-casein with rennet or pepsin and are contained in cheese whey.

Hitherto, kappa-casein glycomacropeptides have been experimentally produced by a method comprising dissolving kappa-casein isolated from cow's milk in deionized water, reacting the solution obtained with pepsin, adding trichloroacetic acid to the solution to precipitate a para-kappa-casein fraction, dialyzing the supernatant obtained against deionized water for desalting, and freeze-drying the solution obtained (Bulletin of Experimental Biology and Medicine, 96, 889 (1983)). Another method comprises dissolving the above kappa-casein in deionized water, adjusting the pH of the solution to 6.7, reacting the solution with rennet, readjusting the pH to 4.6 to precipitate para-kappa-casein, removing the precipitate, dialyzing the supernatant obtained to effect desalting, and freeze-drying the solution obtained (Milk Protein, Academic Press Company, pp 200).

However, the methods conducted in laboratories are not suitable for mass production.

On the other hand, since the industrial utilization of kappa-casein glycomacropeptides has been unknown, the method for producing such compounds in large quantities has not been studied.

Lately, since it was reported that by taking kappa-casein glycomacropeptides dogs lost their appetite (Bulletin of Experimental Biology and Medicine, 96, 889 (1983)), it was found that the compounds were utilizable as food materials for prevention of fat.

Further, since it was found that kappa-casein glycomacropeptides were effective to prevent the adhesion of E.coli to cells of the intestines, to protect the infection of influenza (Japanese Unexamined Patent Application No. 63-284133/1988) or to protect the adhesion of tartar to teeth (Japanese Unexamined Patent Application No. 63-233911/1988), the production of these compounds on a large industrial scale is expected.

Under such conditions, a process for preparing a kappa-casein glycomacropeptide from rennet casein curd whey was reported (Japanese Unexamined Patent Application No. 63-284199). Since the reaction of the process may be carried out without trichloroacetic acid which was used hitherto, the compounds obtained can be used as food materials and can be produced in large quantities. However, the process cannot utilize the rennet casein curds which are obtained as a by-product of the rennet casein curd whey.

If a way of utilisation of the rennet casein curds is not found, the production cost of the kappa-casein glycomacropeptides would be expensive.

In document EP-A-0 291 264 a process for the efficient production of x-casein GMP on an industrial scale, in high purity, is realized by using as a starting effluent an effluent of rennet-casein curd, free of whey proteins, lactose and the like. The pH of the effluent is set to an acidic range to precipitate calcium phosphate and then after removing the precipitate the resulting effluent is subjected to a desalting treatment.

The present inventors have found that the molecular weight of the kappa-casein glycomacropeptides sharply changes at pH 4.

Accordingly, an object of the present invention is to provide a simplified process for producing kappa-casein glycomacropeptides at a low cost by using the above change of the molecular weight at pH 4.

The present invention provides a process for producing a kappa-casein glycomacropeptide, characterised in that the process comprises adjusting the pH of a solution of milk starting materials such as cheese whey, whey protein concentrate and cheese whey from which protein has been removed to below 4, treating the solution by ultrafiltration with a membrane passing a molecular weight fraction of 10,000-50,000 to obtain the filtrate of the solution, preferably readjusting the pH of the filtrate to 4 or higher, and concentrating the filtrate obtained with a membrane passing a molecular weight fraction of 50,000 or less to obtain the desalted concentrate.

It is known that the kappa-casein glycomacropeptides are liberated from casein in the production of cheese. As raw materials, cheese whey, whey protein concentrate which is produced by either ultrafiltration, ion-exchange chromatography or gel filtration, cheese whey from which a whey protein precipitate has been removed by heat treatment or lactose mother liquor can be used in the process of the present invention. When the whey protein concentrate is used, it is diluted with water so as to obtain a preferred concentration of 20% or less by weight. When the concentration is too high, the flow rate of the ultrafiltration is lowered. Any kind of cheese whey can be used. Since small quantities of casein curds and fatty contents are retained in the cheese whey, these are previously removed with a centrifuge, a cream separator or a clarifier. When lactose principally contained in the cheese whey from which protein has been removed by heating is precipitated by the above treatment, the precipitated lactose is removed, for example with a centrifuge or a clarifier or by decantation, and the cheese whey is warmed.

Then, the pH value of the whey is adjusted to 4 or lower, preferably to 3.5 ± 0.2. When the pH value is 4 or higher, since the molecules of the kappa-casein glycomacropeptide are associated, the molecular weight becomes greater and these molecules pass through the membrane with difficulty. The lower limit of the pH value is particularly not limited. When the pH value is 3 or lower, sialic acid which is bonded to the kappa-casein glycomacropeptide becomes unstable, so that the physiological effect of the compound lowers. However, when the kappa-casein glycomacropeptide having no sialic acid is required, the pH value may be 3 or lower. The pH adjustment can be conducted by using an acid. Of such an acid, hydrochloric acid, sulphuric acid, acetic acid, lactic acid, citric acid, are examples.

After adjusting the pH values, the solution is ultra-filtered. The molecular weight fraction of the membrane used in the ultrafiltration stage is 10,000 to 50,000. When a membrane passing a molecular weight fraction below 10,000 is used, the molecules of the kappa-casein glycomacropeptide pass through the membrane with difficulty. When a membrane passing a molecular weight fraction above 50,000 is used, the molecules of the kappa-casein glycomacropeptide can pass through the membrane and a part of the coexisting whey protein can also pass through the membrane, so that the purity of the kappa-casein glycomacropeptide becomes lower. Usually, in the production process of whey protein concentrate, the whey protein is ultrafiltered with a module equipped with a membrane passing a molecular weight fraction of around 20,000. In the process of the present invention, the above membrane can be used.

In the ultrafiltration stage, the solution is preferably concentrated up to the limit and the yield rate of the filtrate is improved.

It is also preferred that water is added to the concentrated solution and the ultrafiltration is conducted repeatedly. For elevating the flow rate of the filtrate, the solution may be heated at about 50°C. However, when the temperature is above 60°C, the whey protein is precipitated or gels, so that the solution is preferably heated to 60°C or less. The concentrate obtained is dried after adjusting the pH value to neutral to obtain the powder of whey protein concentrate.

The filtrate obtained by such a process contains the kappa-casein glycomacropeptide, lactose and minerals. Since the concentration of kappa-casein glycomacropeptide is lowered, the filtrate should be desalted and concentrated. There are two methods of the desalting and the concentration as follows.

In the first method, after readjusting the pH of the filtrate to 4 or higher, the filtrate is filtered with a membrane passing a molecular weight fraction of 50,000 or less by ultrafiltration, diafiltration or reverse osmosis hyperfiltration. The pH of the filtrate can be adjusted by an alkali such as, for example, sodium hydroxide, sodium bicarbonate or ammonia. The monomer of the kappa-casein glycomacropeptide (molecular weight 9,000) is obtained at the pH value 4 or lower, and the polymer of kappa-casein glycomacropeptide (molecular weight 45,000) is obtained at the pH value above 4. If desired, the pH value is adjusted to 5 or higher. Further, any membrane passing a molecular weight fraction of 50,000 or less can be used in the process of the present invention. When the membrane passes a molecular weight fraction above 50,000, the kappa-casein glycomacropeptide can pass through the membrane. The membrane passing a molecular weight fraction about 20,000 which is used in the usual process of producing the concentrate of whey protein can be conveniently used.

When the pH of the filtrate is not readjusted to 4 or higher, the kappa-casein glycomacropeptide exists in the monomer form. Then, the second method is used. In this method, the concentrate is obtained by means of a membrane passing a molecular weight fraction of 10,000 or less, preferably 8,000 or less by ultrafiltration, diafiltration or reverse osmosis hyperfiltration.

These methods can be combined with a desalting process, e.g. by means of electrodialysis or by using an ion exchange resin.

Since the concentrate obtained contains substantially only the kappa-casein glycomacropeptide, it can be dried by spray drying or freeze-drying. Furthermore, since the kappa-casein glycomacropeptide is stable to heat, it is preferred to add a pasteurising or a sterilising step prior to the drying step.

As described above, according to the present invention, the pH value of the solution of milk starting materials such as cheese whey, whey protein concentrate and cheese whey from which protein has been removed is adjusted. Such a simple operation can provide a process of producing kappa-casein glycomacropeptides on a large scale and at a low cost. The product obtained can have high purity.

Moreover, in a factory where the whey protein concentrate has been produced, a new equipment is not required because kappa-casein glycomacropeptides can be produced with the ultrafilter or the reverse osmosis hyperfilter for preparing the whey protein concentrate. The protein fraction from which the kappa-casein glycomacropeptides has been removed can be used as the whey protein concentrate.

Further, the products can be obtained without using additives such as trichloroacetic acid. Accordingly, the kappa-casein glycomocropeptides obtained can be used as raw materials in the fields of food and medical supplies. It is very useful in industrial fields.

The following Examples illustrate the present invention more specifically.

### Example 1

### Preparation of a kappa-casein glycomacropeptide from whey protein concentrate

One kg of whey protein concentrate (manufactured by Taiyo Kagaku Ltd. Trade name: Sunlact N-2) was dissolved in 50 liters of water at 50°C and the pH value was adjusted to 3.5 by using 12 N hydrochloric acid. The solution was filtered with an ultrafiltration membrane passing a molecular weight fraction of 20,000 (manufactured by DDS Ltd., GR 61PP) at 50°C, at a pressure of 0.4 MPa and an average permeation flux of 52.4 l/m²·h. When the filtrate volume reached 40 liters, 40 liters of water heated at 50°C was added to the concentrated solution and the ultrafiltration of the concentrated solution was successively conducted. By the successive operations, the amount of the filtrate obtained was 160 liters.

To the filtrate obtained, 25% sodium hydroxide was added to obtain a solution having pH 7.0. Again, using the same ultrafiltration membrane under the same conditions, the filtrate was concentrated and 5 liters of the desalted concentrate was obtained. Then, water heated to 50°C was added to the concentrated solution to maintain a total mount of 10 liters, the concentrated solution was treated with the same ultrafiltration membrane under the same conditions by diafiltration to desalt the concentrate. When the filtrate volume reached 80 liters, the addition of water to the concentrated solution was stopped. When the volume of the concentrated solution was reduced to two liters by ultrafiltration, the concentrated solution was freeze-dried. 54 g of the kappa-casein glycomacropeptide was obtained. The purity 82% of the product was determined by an urea-SDS electrophoresis method. (In the urea-SDS electrophoresis method, a sample was electrophoresed and dyed by using Coomassie brilliant blue. The peak area of the obtained dyed gel was measured with a densitometer (GELMAN ACD-12) to determine the GMP purity of the sample.)

### Example 2

### Preparation of kappa-casein glycomacropeptide from cheese whey.

The pH value of 500 liters of Gouda cheese whey (manufactured by SNOW BRAND MILK PRODUCTS CO., LTD.) was adjusted to 3.5 by using concentrated hydrochloric acid and insolubles were removed with a clarifier. The whey solution was heated to 50°C by using a plate heater. Using the same conditions as in Example 1, the solution was concentrated to obtain a solid content of 20%. To 360 liters of the filtrate obtained, an aqueous solution of 25% sodium hydroxide was added to obtain a solution having pH 7.0. Using the same method as in Example 1, the solution was concentrated, desalted and freeze-dried. 5.7 g of the kappa-casein glycomacropeptide was obtained. A purity 80% of the product was determined by an urea-SDS electrophoresis method.

### Example 3

### Preparation of a kappa-casein glycomacropeptide from cheese whey excluding protein.

The pH value of 500 liters of Gouda cheese whey (manufactured by SNOW BRAND MILK PRODUCTS CO., LTD.) was adjusted to 4.6 by using concentrated hydrochloric acid and the protein was precipitated, and then supernatant was obtained by decantation. The supernatant obtained was heated at 80°C for 10 minutes, and insolubles were removed with a clarifier. After cooling to 50°C, the pH value of the supernatant was adjusted to 3.5 by using concentrated hydrochloric acid. Maintaining the temperature of 50°C and using the same conditions as in Example 2, the concentrated solution was concentrated to obtain a solid content of 20%. To 410 liters of the filtrate obtained, an aqueous solution of 25% sodium hydroxide was added to obtain a solution having pH 7.0. Using the same method as in Example 1, the solution was concentrated, desalted and freeze-dried. 6.5 g of the kappa-casein glycomacropeptide was obtained. The purity 78% of the product was determined by an urea-SDS electrophoresis method.

### Example 4

### Preparation of a kappa-casein glycomacropeptide from whey protein concentrate and desalting and concentration of the peptide with a loose RO membrane.

One kg of whey protein concentrate (manufactured by Taiyo Kagaku Company, Trade name: Sunlact N-2) was dissolved in 50 liters of water at 50°C. Using the same method as in Example 1, the solution was ultrafiltered and 160 liters of filtrate was obtained. The pH value of the filtrate was held at 3.5. The filtrate was filtered with an ultrafiltration membrane passing a molecular weight fraction of 5,000 (manufactured by DDS Company, GR 81PP) at 50°C, at a pressure of 0.4 MPa and an average permeation flux of 38.8 l/m²·h. When the volume of the concentrated solution was reduced to two liters by desalting and concentration, the concentrated solution was freeze-dried. 54 g of the kappa-casein glycomacropeptide was obtained. The purity 80% of the product was determined by an urea-SDS electrophoresis method.

## Claims

1. A process for producing a kappa-casein glycomacropeptide which comprises adjusting the pH of a solution of milk starting materials containing the kappa-casein glycomacropeptide to below 4, treating the solution by ultrafiltration with a membrane passing a molecular weight fraction of 10,000-50,000, and concentrating the filtrate obtained with a membrane passing a molecular weight fraction of 50,000 or less.

2. A process as claimed in claim 1, wherein the pH value of the filtrate obtained by the ultrafiltration treatment is readjusted to 4 or higher and then the filtrate is concentrated.

3. A process as claimed in claim 1 or 2, wherein the concentration of the filtrate is carried out with a membrane passing a molecular weight fraction of 10,000 or less.

## Patentansprüche

1. Verfahren zur Herstellung eines kappa-Kasein-Glycomakropeptides, wobei der pH-Wert einer, die kappa-Kassein-Glycomakropeptide enthaltenden Lösung aus Milchausgangsstoffen auf weniger als 4 eingestellt wird, wobei die Lösung einer Ultrafiltration unter Verwendung einer für eine Molekulargewichtsfraktion von 10 000 bis 50 000 durchlässigen Membran unterzogen wird und wobei das erhaltene Filtrat unter Verwendung einer für eine Molekülargewichtsfraktion von 50 000 oder weniger durchlässigen Membran konzentriert wird.

2. Verfahren nach Anspruch 1, dadurch gekennzeichnet, daß der pH-Wert des durch Ultrafiltration erhaltenen Filtrates bei 4 oder einem höheren Wert eingestellt wird und daß das Filtrat anschließend konzentriert wird.

3. Verfahren nach Anspruch 1 oder 2, dadurch gekennzeichnet, daß das Filtrat unter Verwendung einer für eine Molekulargewichtsfraktion von 10 000 oder weniger durchlässigen Membran konzentriert wird.

## Revendications

1. Procédé de préparation d'un glycomacropeptide de kappa-caséine, comprenant les opérations consistant à régler à moins de 4 le pH d'une solution de substances de départ provenant du lait et contenant le glycomacropeptide de kappa-caséine, à traiter la solution par ultrafiltration avec une membrane laissant passer une fraction de poids moléculaire de 10 000 à 50 000, et à concentrer le filtrat obtenu avec une membrane laissant passer une fraction de poids moléculaire de 50 000 ou moins.

2. Procédé selon la revendication 1, dans lequel le pH du filtrat obtenu par le traitement d'ultrafiltration est réajusté à 4 ou à une valeur supérieure, puis le filtrat est concentré.

3. Procédé selon la revendication 1 ou 2, dans lequel la concentration du filtrat est effectuée avec une membrane laissant passer une fraction de poids moléculaire de 10 000 ou moins.
